# EUROPÄISCHE PATENTANMELDUNG

(11) **EP 1 421 858 A1**
(43) Veröffentlichungstag der Anmeldung: **26.05.2004**
(21) Anmeldenummer: 03026659.7
(22) Anmeldetag: 19.11.2003
(51) Int. Cl.: A23L 1/30, A23L 1/29, A23L 1/03, A61K 31/20

(54) **Verwendung von mittelkettigen Triglyceriden (MCT) zur ernährungsphysiologischen Optimierung des Fettsäurespektrums in einem diätischen Lebensmittel für Diabetiker**

(30) Priorität: 22.11.2002 DE 10254584
(71) Anmelder: HORST HEIRLER PROJEKTE ERNÄHRUNG*MEDIZIN*ÖKOLOGIE, 82234 Oberpfaffenhofen (DE)
(72) Erfinder: Heirler, Horst, 82237 Wörthsee (DE)
(74) Vertreter: VOSSIUS & PARTNER

(57) **Zusammenfassung**

Beschrieben wird die Verwendung von MCT bzw. eines diese enthaltenden diätetischen Lebensmittels für besondere medizinische Zwecke zur ergänzenden bilanzierten Ernährung/diätetischen Behandlung von Erwachsenen und Heranwachsenden mit Diabetes mellitus (Zuckerkrankheit).

## Beschreibung

Die vorliegende Erfindung betrifft die Verwendung von MCT bzw. eines diese enthaltenden diätetischen Lebensmittels für besondere medizinische Zwecke zur ergänzenden bilanzierten Ernährung/diätetischen Behandlung von Erwachsenen und Heranwachsenden mit Diabetes mellitus (Zuckerkrankheit).

Beim Diabetes mellitus handelt es sich um eine Gruppe von Erkrankungen, die mit einer chronische Regulationsstörung des Stoffwechsels einhergehen. Charakteristisch ist eine chronischen Blutzuckerhöhung (Hyperglykämie), die aus einem Defekt in der Insulinsekretion oder der Insulinwirkung resultiert. Die zwei Haupttypen sind der so genannte Typ-1-Diabetes, der durch einen absoluten Insulinmangel bedingt ist, und der so genannte Typ-2-Diabetes, für den eine verringerte Sekretion und/oder eine erniedrigte Insulinempfindlichkeit der peripheren Gewebe charakteristisch sind. Die Häufigkeit beider Typen steht in einem Verhältnis von etwa 1 : 9.

Klinisch bedeutsam ist, dass die chronische Hyperglykämie beim Diabetes mellitus zu Gefäßveränderungen in Form der Makro- und Mikroangiopathie sowie zu Nervenschädigungen führt. Diese Folgeerkrankungen werden durch langfristige Organbeeinträchtigungen, vor allem der Blutgefäße, des Herzens, der Nieren, der Augen und der Nerven, hervorgerufen. Zentrale Bedeutung kommt bei der Entstehung einer diabetischen Makro- und Mikroangiopathie den durch hohe Glukosekonzentrationen bedingten irreversiblen Veränderungen langlebiger Moleküle in Zellen zu, insbesondere in Zellkernen. Dem Mechanismus solcher Beeinträchtigungen liegt die nichtenzymatische Bildung von stabilen Glykosilierungsprodukten ("advanced glycosylation products") zugrunde. Diese sind vom Ausmaß und von der Dauer der Glukoseanreicherung abhängig und wirken im Kontext und Wechselspiel mit anderen aggressiven Faktoren wie freien Radikalen und/oder Lipidperoxidationsprodukten.

Die chronische Regulationsstörung betrifft nicht nur den Kohlenhydrat-, sondern auch den Fett-, Eiweiß- und Elektrolytstoffwechsel. Die Hyperglykämie führt bei Überschreiten der Nierenschwelle für Glukose zur Glukosurie, zu einem Glukosemangel im Muskel- und Fettgewebe sowie zu einer Steigerung der Glukoneogenese. Die Störung des Fettstoffwechsels zieht eine eingeschränkte Synthese und Speicherung von Neutralfetten nach sich. Demzufolge werden die Mobilisation von Depotfetten stimuliert und der metabolische Fettumsatz gesteigert. Die vermehrte Bildung von Azetessigsäure und Beta-Hydroxybuttersäure verursacht eine Ketose, und es kommt zu erhöhtem oxidativen Stress. Da auch die Bildung von Lipoproteinen intensiviert, deren Abbau jedoch verzögert ist, stellt sich eine, durch peroxidativ veränderte Lipoproteine bedingte Dyslipoproteinämie ein. Der gestörte Proteinstoffwechsel ist durch eine Hemmung der Proteinsynthese und einen verstärkten Proteinabbau gekennzeichnet. Die vermehrt frei gesetzten Aminosäuren gehen in die Glukoneogenese und den Energiestoffwechsel ein. Bei Insulinmangel ist überdies der Elektrolytstoffwechsel gestört. Die Folgen sind ein zellulärer Kaliumverlust und sekundäre Aufhebungen des Wasser-, Natrium- und Säure-Basen-Gleichgewichts.

Während sich die gefürchteten akuten Stoffwechselentgleisungen (Coma diabeticum, Coma hypoglycaemicum) inzwischen gut beherrschen lassen, sind die angeführten, durch den Diabetes mellitus hervorgerufenen chronischen Folgekrankheiten und Spätschäden in den Vordergrund der Gesundheitsprobleme von Diabetikern gerückt.

Die ernährungsmedizinische Behandlung stellt eine integrale Säule der Diabetestherapie dar. Ziele dieser Therapie sind : (a) die Normalisierung des Blutglukosespiegels, um das Risiko für Komplikationen, insbesondere Folgekrankheiten, zu reduzieren oder es sogar zu verhindern; (b) ein Lipid- und Lipoproteinprofil, welches das Risiko für eine Makroangiopathie verringert, und (c) das Erreichen von Blutdruckwerten, die das Risiko für Gefäßerkrankungen herabsetzen. Die metabolischen Konditionen dafür können und sollten durch eine Lebensstiländerung, insbesondere eine modifizierte Ernährung erlangt werden. Sie eignen sich zur Prävention und Behandlung von Adipositas, Hyperlipoproteinämie, kardiovaskulären Erkrankungen, Bluthochdruck und Nierenerkrankungen.

Für den Diabetiker ist eine optimale Stoffwechselkontrolle die unbestritten beste Prävention und Therapie. Sie schützt vor Makro- und Mikroangiopathie bzw. reduziert das Risiko dafür, und das gilt ebenso für andere Komplikationen und Folgeschäden. Dabei spielt die Ernährung bei Insulinresistenz und Typ-2-Diabetes insbesondere im Rahmen des "Metabolischen Syndroms" eine Rolle. Dieses geht mit Insulinresistenz, Adipositas, kardiovaskulären Erkrankungen, Hypertonie und Dyslipoproteinämie einher. Etwa 80 % der Typ-2-Diabetiker sind nämlich übergewichtig. Abdominelle Fettsucht, körperliche Inaktivität, Dyslipoproteinämie und gestörte Glukosetoleranz sind die vornehmlichen Ursachen einer Manifestation des Diabetes. Das Metabolische Syndrom gilt deshalb als das klassische Risiko für die Ausbildung einer vorzeitigen Arteriosklerose.

Die ernährungstherapeutischen und -präventiven Ansatzpunkte für die Verzögerung und - besser noch - für die Vermeidung der aufgeführten Spätkomplikationen liefern, wie bei der Arteriosklerosevorbeugung schlechthin, die Regulierung und Normalisierung des Lipidstoffwechsels. Dabei ist vorzugsweise von folgenden Risikofaktoren auszugehen : (a) Übergewicht, insbesondere abdominelle Adipositas, infolge einer zu hohen Nahrungsenergie- und -fettzufuhr, (b) Verzehr von langkettigen gesättigten und von trans-ungesättigten Fettsäuren, (c) darüber hinaus ein physiologisch und für die Hämodynamik ungünstiges Verhältnis von Alkan-, Mono- und Polyalkenfettsäuren in der Nahrung, (d) eine Relation von Omega-6- zu Omega-3-Nahrungsfettsäuren > 5 : 1, (e) eine unzureichende Versorgung mit langkettigen Omega-3 Fettsäuren (C20 : 5 = Eicosapentaensäure und C22 : 6 = Docosahexaensäure), (f) eine ungenügende Verhinderung der Peroxidation von polyalkenreichen Lipiden der Nahrung und demzufolge in vivo auch der LDL (Low Density Lipoproteins) wegen einer unzureichenden exogenen Bereitstellung antioxidativer Schutzstoffe, speziell der Vitamine E und C sowie (g) eine unzureichende Zufuhr pflanzlicher Sterole.

Ziel der Ernährungstherapie bei Diabetes ist es deshalb, mit einer Normoglykämie die genannten Risiken für atherosklerotische Gefäßerkrankungungen zu reduzieren oder sogar zu verhindern. Nach allgemeiner Übereinkunft und ebenso gemäß den Empfehlungen für die Ernährung von Diabetikern, welche die Diabetes and Nutrition Group (DNSG) of the European Association for the Study of Diabetes (EAS) und der Ausschuss Ernährung der Deutschen Diabetes-Gesellschaft (DDG) 2000 sowie in einem Positionspapier die American Diabetes Association (ADA) 2002, herausgegeben haben, bedeutet dies, durch Verringerung der aufgenommenen Nahrungsfett-Energie auf 25 bis 35 % der Gesamtenergie einen Body- Mass-Index von 18,5 bis 25 kg/m² anzustreben, 60 bis 70 % der Nahrungsenergie-% durch Kohlenhydrate und einfach ungesättigte Fettsäuren mit cis-Konfiguration zu bestreiten und die Verteilung der Nahrungsfett-Energie folgendermaßen zu gestalten : (1) gesättigte, speziell langkettige Fettsäuren + trans-ungesättigte Fettsäuren : < 8 % (ADA < 7 %), (2) cis-einfach ungesättigte Fettsäuren (hauptsächlich Ölsäure) :10 bis 20%, (3) mehrfach ungesättigte Fettsäuren (Polyalkenfettsäuren, PUFA) : ≤ 10%, (4) ein Verhältnis von Omega-6- : Omega-3-Fettsäuren ≤ 5 : 1, (5) langkettige Polyalkenfettsäuren (Fischölfettsäuren; C20 : 5 = Eicosapentaensäure; C22 : 6 = Docosahexaensäure) : ≥ 0,2% sowie (6) Förderung und zweckbestimmte Steigerung der Aufnahme antioxidativer Nahrungsinhaltsstoffe (Nahrungsantioxidanzien). Außerdem ist bekannt, dass der Verzehr einfach ungesättigter Fettsäuren in Mengen ≥ 15 Nahrungsenergie-% einen erhöhten Lipidserumspiegel senkt.

Eine solche Empfehlung für Diabetiker entspricht weitgehend der für die Normalbevölkerung, wie sie die Ernährungsgesellschaften Deutschlands, Österreichs und der Schweiz in ihren D-A-CH-Referenzwerten von 2000 zum Ausdruck gebracht haben. Eine etwas andere Position vertritt insofern die DGG, als sie die Aufnahme an Energie aus Nahrungsfetten liberaler sieht, weil sie die Qualität der zugeführten Fettsäuren für entscheidender hält und stark auf einen niedrigen glykämischen Index von Lebensmitteln abhebt. Tatsächlich bestreiten im Durchschnitt der Bevölkerung (gemäß dem Ernährungsbericht 2000 der Deutschen Gesellschaft für Ernährung) Nahrungsfette noch immer rund 36 % der Nahrungsenergie und Kohlenhydrate lediglich 44 bis 46 %. Gesättigte Fettsäuren liefern knapp 15 % der Nahrungsenergie, einfach ungesättigte etwa 13,5 % und mehrfach ungesättigte 5,7 %. Das Verhältnis von Omega-6- zu Omega-3-Fettsäuren beträgt 8 : 1. Die maximal aus der durchschnittlich aufgenommenen Menge an alpha-Linolensäure (C18 : 3 Omega-3) gebildete Menge an Fischölfettsäuren beläuft sich auf 0,15 g/Tag. Veranschlagt man die beim Fischverzehr selbst zugeführte Menge mit 0,1 g/Tag, so ergibt sich eine tägliche Versorgung mit 0,25 g Eicosapentaen- + Docosahexaensäure. Das entspricht nur etwa 0,1% der Nahrungsenergiezufuhr und nicht, wie empfohlen, > 0,2 Energie-%. Hinzu kommt, dass (nach den 2002 veröffentlichten Ergebnissen einer repräsentativen Erhebung des Berliner Robert-Koch-Institutes) ein erheblicher Teil der (deutschen) Bevölkerung die D-A-CH-Referenzwerte für Vitamine nicht erfüllt. Für die antioxidativen Vitamine E und C sind es z. B. rund 60 und 24 %.

Die bei Diabetikern für notwendig gehaltene Förderung und zweckbestimmte Steigerung der Aufnahme antioxidativer Nahrungsinhaltsstoffe besitzt demzufolge keine gute Ausgangslage. In Anbetracht dieser allgemeinen Ernährungssituation kommt es also darauf an, bei der Veränderung des Nahrungsfettverzehrs vor allem die Aufnahme an gesättigten Fettsäuren zu reduzieren, die an trans-Fettsäuren möglichst auszuschließen und die an ungesättigten anzuheben, und zwar zu Gunsten der Omega-3- und zu Lasten der Omega-6-Fettsäuren.Darüber hinaus ergibt sich die Notwendigkeit, den Verzehr an Nahrungsantioxidanzien merklich anzuheben.

In der Ernährungspraxis erfolgt die unkontrollierte Veränderung des Nahrungsfettverzehrs mehr durch Weglassen von Streich- und Bratfetten sowie von Koch- und Salatölen als durch Herabsetzen des Verzehrs von Lebensmitteln tierischer Herkunft sowie von gebackenen, fritierten, gebratenen oder mit fetthaltigen Überzügen versehenen Erzeugnissen mit hohen Anteilen an gesättigten Fettsäuren in den so genannten versteckten Fetten. Darum und weil es weder Margarinen und andere Streichfette noch Backfette, Salat-, Koch- und Bratöle mit der Zusammensetzung gibt, die den vorgenannten Empfehlungen gerecht wird, besteht bei Diabetikern immer die Gefahr, dass es bei Beibehaltung oder Drosselung ihres Fettverzehrs zu einem Fettsäurenmuster in der Nahrung kommt, welches dem empfohlenen nicht entspricht und das demzufolge einer Regulierung und Normalisierung des Lipidstoffwechsels im Sinne der Arteriosklerose-Prävention ― im Organismus über die Fettsäurenzusammensetzung der Lipoprotein- und Membranphospholipide ― entgegen steht. Es sind zwar Margarinen mit und ohne Fettreduktion (80 und 60 % Fett) bekannt, die neben Ölsäure (C18 : 1 Omega-9) und den essentiellen Fettsäuren Linolsäure (C18 : 2 Omega-6) und alpha-Linolensäure (C18 : 3 Omega-3) auch schon Eicosapentaensäure (C20 : 5 Omega-3) und Docosahexaensäure (C22 : 6 Omega-3) in einem physiologisch angemessenen Verhältnis aufweisen. Sie sind wegen der Verwendung gehärteter Fette als Festbestandteile aber mit dem Nachteil des Gehalts an langkettigen sowie an ungesättigten Trans-Fettsäuren behaftet. Außerdem reichen die ihnen zugesetzten Vitamine in ihrer Menge und Konzentration bestenfalls zum Oxidationsschutz der in ihnen als Nahrungskomponenten enthaltenen ungesättigten Fettsäuren aus.

Der vorliegenden Erfindung liegt somit das technische Problem zugrunde, ein diätetisches Lebensmittel für Patienten mit Diabetes mellitus zu schaffen, das diese Nachteile nicht aufweist, damit krankhafte Veränderungen des Fettstoffwechsels ernährungstherapeutisch zu behandeln und so den Fettstoffwechsel zu regulieren und zu normalisieren hilft und auf diese Weise die Lebensqualität einschränkenden und am Ende lebensbedrohenden Folgeerkrankungen vorbeugt, vorzugsweise Makro- und Mikroangiopathien.

Die Lösung dieses Problems erfolgt durch die Gewährleistung der in den Ansprüchen gekennzeichneten Ausführungsformen. Es wurde nämlich überraschenderweise gefunden, dass sich als Festfette für die Herstellung von Streichfetten (Margarinen) und Backfetten, die den dargelegten ernährungsmedizinischen Ansprüchen genügen, mittelkettige Triglyceride (MCT) eignen und dass sich diese Eignung nicht nur aus technologischer sondern auch aus ernährungsphysiologischer und -medizinischer Sicht ergibt. Die erfindungsgemäße Zusammensetzung bzw. das erfindungsgemäße diätetische Lebensmittel erreicht als Streich- und Backfett sowie als Öl erstmals eine Zusammensetzung und ein Verhältnis der Fettsäuren, wie sie für das Ernährungsregime von Diabetikern vom Grundsatz her gefordert werden müssen.

Zusammengefasst besteht die erfinderische Leistung vor allem darin, dass (a) erstmals mittelkettige Triglyceride (MCT) bei der ergänzenden bilanzierten diätetischen Behandlung von Diabetikern zur Regulierung und zur Optimierung der Stoffwechselsituation eingesetzt und (b) diese vorzugsweise in Kombination mit ungesättigten Fettsäuren, insbesondere den langkettigen Omega-3-Fettsäuren, für die besonderen Stoffwechselbedürfnisse von Diabetikern als ergänzende bilanzierte Diät in Form von Streichfetten und/oder Speiseölen formuliert werden.

Die Verwendung eines bestimmten Prozentsatzes solcher mittelkettigen Triglyceride, vorzugsweise mit einem nahezu ausschließlichen Gehalt an Caprylsäure (C8 : 0) und/oder Caprinsäure (C10 : 0), nachstehend MCT genannt, hat in Kombination mit Ölsäure (C18 : 1) und insbesondere mit langkettigen (LCT) Omega-3-Fettsäuren aus Fischölen folgende, entscheidende Vorteile : (a) MCT gelangen unter Umgehung des Lymphweges über die Pfortader direkt in die Leber und werden dort oxidiert, (b) MCT werden nicht im Fettgewebe gespeichert, (c) MCT haben mit 8,3 kcal/g Fett einen niedrigeren Brennwert als langkettige Fettsäuren mit 9,3 kcal/g Fett.

Allein dadurch werden schon eine Reduzierung der Aufnahme von Fettenergie erreicht und erforderliche Körpergewichtsabnahmen begünstigt. Der Einsatz eines relativ hohen Anteils von Ölsäure (C18 : 1 Omega-9) verbessert bereits die Flexibilität und die Verformbarkeit der phospholipidhaltigen Membranen von Blutzellen und damit die Hämodynamik bei gleichzeitiger Verringerung des Risikos fataler Gerinnungsvorgänge. Die Verwendung eines hochgereinigten Konzentrates von Omega-3-Fischöl-Fettsäuren (C20 : 5, C22 : 6) hebt den Nachteil der mit pflanzlichen Ölen eingebrachten Omega-3-Fettsäure alpha-Linolensäure (C18 : 3) auf, nämlich endogen gewöhnlich nur zu 2 bis 7 % und in Ausnahmen bis zu maximal 10% in die LCT-Omega-3-Fettsäuren (C20 : 5 und C22 : 6) umgewandelt zu werden und demzufolge die erforderliche Versorgung mit diesen nicht zu erbringen.

Durch die erfindungsgemäß erzielte Veränderung des Fettsäuren-Spektrums kommt es beim Diabetiker zu einer umfassenden Beeinflussung der metabolischen Situation. Dabei spielt auch die Veränderung der Eicosanoid-Synthese im Organismus eine wesentliche Rolle. Denn infolge vermehrter Bildung von Eicosanoiden aus Eisosapentaensäure (C20 : 5 Omega-3) anstatt aus Arachidonsäure (C20 : 4 Omega-6; diese wird sowohl mit Lebensmitteln tierischer Herkunft verzehrt als auch im menschlichen Organismus aus Linolsäure [C18 : 2 Omega-6] gebildet), kommt es zu einer Reduzierung des atherosklerotischen Risikopotenzials. Das diesbezügliche Risikopotenzial ergibt sich aus einer gesteigerten Thrombozytenaggregation und -adhäsion, einer erhöhten Vasokonstriktion sowie aus vermehrten Entzündungsreaktionen.

Somit betrifft die vorliegende Erfindung die Verwendung von mittelkettigen Triglyceriden (MCT) und/oder einer mittelkettige Triglyceride enthaltenden Zusammensetzung zur diätetischen Behandlung von Personen mit Diabetes mellitus.

Der hier verwendete Ausdruck "mittelkettige Triglyceride" bezieht sich auf Triglyceride mit einem nahezu ausschließlichen Gehalt an Octansäure (Caprylsäure; C8 : 0) und/oder Dekansäure (Caprinsäure; C10 : 0) entsprechend den systematischen und trivialen Namen in der chemischen Nomenklatur.

Der Fachmann kennt als Quellen für diese mittelkettigen Triglyceride vorzugsweise Kokosnuss- und Palmkernfett.

Die ausschließliche Verwendung von MCT-Fetten als Kostbestandteil ist begrenzt. Daher empfiehlt sich die Zufuhr von MCT in Form MCT-haltiger Lebensmittel, z. B. Margarinen, so dass sich für Diabetiker viele Möglichkeiten ergeben, sie zu verzehren. Da bislang pflanzliche Öle und Ölmischungen für Koch- und Bratzwecke sowie für das Anrichten von Salaten und die Zubereitung von anderen Speisen mit dem für Diabetiker wünschenswerten Fettsäuren-Spektrum gleichfalls nicht verfügbar sind, ist es vorteilhaft, das Prinzip der erfindungsgemäßen Problemlösung ebenso auf Salat- sowie Koch- und Bratöle zu übertragen.

In einer bevorzugten Ausführungsform enthält das erfindungsgemäße diätetische Lebensmittel als Fett/Öl-Gemisch deshalb vergleichbare Fettsäuren-Zusammensetzungen und -Verhältnisse wie die Fettphasen von Margarinen und anderen Streichfetten mit dem vorstehend begründeten ernährungsphysiologischen und -medizinischen Anspruch einer besonderen Eignung für Ernährungsregime von Diabetikern. Es werden in diesem Fall lediglich die langkettigen Fischölfettsäuren nicht berücksichtigt, weil sie hitzeempfindlich und besonders oxidationsanfällig sind, was sich auf damit zubereitete Speisen geschmacklich nachteilig auswirken könnte.

Bei dieser Ausführungsform sind jedoch MCT gleichfalls ein integraler Bestandteil von Speiseölen für die küchentechnische Zubereitung von Salaten, Suppen bzw. Eintöpfen, Soßen, Tunken, Dressings, Dips, Ketchups, Chutneys und anderen Würzsoßen, Majonäsen, Remouladen und sämtlichen weiteren Speisen, die unter Verwendung von Pflanzen-, Back-, Brat-, Fritier- und Grillölen bzw. -fetten u. Ä. verzehrsfähig oder -fertig gemacht werden.

Gemäß der Definition und Zweckbestimmung nach der Richtlinie 1999/21/EG vom 25. März 1999 (und deren Umsetzung in deutsches Recht durch die Diätverordnung i. d. F. der Bekanntmachung vom 25. 08. 1988, zuletzt geändert durch Art. 1 zehnte Diät-VO-Änd. vom 21. 12. 2001) handelt es sich bei den erfindungsgemäß und nachfolgend beispielhaft beschriebenen Produkten um diätetisch unvollständige Lebensmittel mit einer für eine bestimmte Krankheit oder Störung spezifisch angepassten Standardformulierung, die sich nicht für die Verwendung als einzige Nahrungsquelle eignen. Ihr besonderer Ernährungszweck ist vielmehr die bilanzierte Ergänzung einer individuellen Basisdiät, welche die glykämische Kontrolle normalisieren und damit Komplikationen und Spätfolgen verhindern und/oder minimieren soll. Dabei muss besonderes Augenmerk außer auf die Relation der Hauptnährstoffe Kohlenhydrate, Fette und Proteine auf die Qualität der Nahrungsfette und das Verhältnis ihrer Fettsäuren zu- und untereinander gerichtet werden. Das ist bei bestimmungsgemäßem Verzehr der erfindungsgemäßen Streichfette (Margarinen) sowie Brat-, Koch- und Salatöle einfach zu erreichen.

In einer bevorzugten erfindungsgemäßen Verwendung enthält die Zusammensetzung in der Fettphase (a) 10 bis 30 % mittelkettige Triglyceride, (b) Monoensäure(n) (einfach ungesättigte Fettsäuren), vorzugsweise Ölsäure (C18 : 1), vorzugsweise 20 bis 60%, und/oder (c) Diensäure(n) (zweifach ungesättigte Fettsäuren) wie Linolsäure (C18 :2), vorzugsweise 10 bis 35%, und/oder (d) Triensäure(n) (dreifach ungesättigte Fettsäuren) wie alpha-Linolensäure, vorzugsweise 3 bis 10%.

Quellen für Ölsäure sind vor allem Olivenöl und Raps- bzw. Canolaöl. Die Linolsäure stammt vorzugsweise aus Sonnenblumen- und aus Rapsöl. Die alpha-Linolensäure wird in erster Linie durch Rapsöl eingetragen, doch Leinöl stellt ebenfalls eine geeignete Quelle dafür dar.

Um einer unzureichenden Versorgung mit langkettigen Polyalkensäuren (mehrfach ungesättigten Fettsäuren; C20:5 = Eicosapentaensäure und C22:6 = Docosahexaensäure) vorzubeugen, enthält in einer besonders bevorzugten erfindungsgemäßen Verwendung die Zusammensetzung in der Fettphase weiterhin Eicosapentaensäure und/oder Docosahexaensäure, vorzugsweise aus Seetierfetten und speziell aus Mischungen hochgereinigter (raffinierter) Fischöle, wobei Werte von 0,5 bis 2% Eicosapentaensäure und/oder Docosahexaensäure in Form von Triglyceriden favorisiert werden.

In einer weiteren bevorzugten erfindungsgemäßen Verwendung ist der Gehalt an gesättigten langkettigen (>12 C-Atome) Fettsäuren (Alkanfettsäuren) in der Zusammensetzung höchstens 6%. Diese können aus Pflanzenfetten wie Oliven-, Raps-, und Sonnenblumenöl oder Kokosfett und/oder Kuhmilchbutter stammen.

In einer noch mehr bevorzugten erfindungsgemäßen Verwendung ist die Zusammensetzung in der Fettphase folgendermaßen :

| | |
|---|---|
| (a) Mittelkettige Triglyceride | 10 bis 30%; |
| (b) Gesättigte langkettige Fettsäuren | 0,5 bis 6%; |
| (c) Ölsäure | 20 bis 60%; |
| (d) Linolsäure | 10 bis 35 %; |
| (e) alpha-Linolensäure | 3 bis 10%; und |
| (f) Eicosapentaen- und/oder Docosahexaensäure | 0,5 bis 2% |

Vorzugsweise enthält die Fettphase der Zusammensetzung für die erfindungsgemäße Verwendung außer den vorstehend beschriebenen Komponenten zusätzlich als Emulgatoren Mono- und Diglyceride von Speisefettsäuren (MDG wie z.B. E 471), aber keine Phosphatide wie Lezithin, die als natürliche Emulgatoren beispielsweise in Form von Nebenprodukten bei der Fettraffination anfallen. Dazu kommen fettlösliche Vitamine, vorzugsweise die Vitamine A, D, E und/oder Vitamin C in Form von Ascorbylpalmitat, β-Carotin, Butter- und/oder - in Hinsicht auf die hochungesättigten Fettsäuren - geeignete würzende Aromen wie z. B. Rosmarinextrakte.

Hinsichtlich der vorstehend aufgeführten fettlöslichen Vitamine sind folgende Konzentrationen in der Fettphase von 100 g des emulgierten Endproduktes besonders bevorzugt: 0,0002 bis 0,002 g Retinylpalmitat (Vitamin A),1 bis 5 µg (40 bis 200 I.E.) Vitamin D₃ (Cholecalciferol), 0,06 bis 0,6 g Ascorbylpalmitat, 0,02 bis 0,2 g RRR-α-Tocopherylacetat (natürliches Vitamin E).

Für die Basisdiät wurde nun überraschend herausgefunden, dass sich mit dem erfindungsgemäßen diätetischen Lebensmittel als ergänzender bilanzierter Diät auch die glykämische Kontrolle erreichen und die Regulation des bei Diabetes mellitus komplex gestörten Stoffwechsels verbessern lässt, indem der wässrigen Phase von Streichfetten (Margarinen) als elementare metabolische Coenzyme bestimmte B-Vitamine beigefügt werden.

In einer bevorzugten Ausführungsform enthält daher z. B. ein diätetisches Streichfett (Margarine) neben den zu den metabolisch elementaren B-Vitaminen gerechneten auch Vitamin B₆, Folsäure und Vitamin B₁₂, weil diese drei B-Vitamine im Homocysteinstoffwechsel eine bedeutende Rolle spielen. Die Hyperhomocysteinämie ist nämlich ein weiterer, eigenständiger Risikofaktor für vaskuläre Erkrankungen als Komplikationen des Diabetes mellitus, der man mit einer guten Versorgung durch diese drei Vitamine begegnen kann.

In der am meisten bevorzugten Verwendung liegen die Vitamine in der Zusammensetzung in der Wasserphase in folgenden Mengen (und bevorzugten Verbindungen) vor: 0,01 bis 0,25 g Vitamin C (als Natriumascorbat); 0,0005 bis 0,005 g Vitamin B₁ (als Thiaminmononitrat); 0,0006 bis 0,006 g Vitamin B₂ (als Riboflavin-5'-Natrium); 0,0007 bis 0,007 g Vitamin B₆ (als Pyridoxinhydrochlorid); 0,0015 bis 0,015 mg B₁₂ (als Cyanocobalamin); 0,007 bis 0,07 g Niacin (als Nikotinamid); 0,0002 bis 0,002 g Folsäure (als Pteroylmonoglutamat).

In einer alternativen Ausführungsform werden anstelle eines hochgereinigten Fischölkonzentrats zur Gewährleistung einer genuinen Versorgung mit Eicosapentaen- und Docosahexaensäure der wässrigen Phase Salze der Spurenelemente Zink, Chrom und/oder Mangan beigefügt.

Als Cofaktoren bzw. Aktivatoren tragen Zink, Chrom und Mangan effektiv zur Regulierung und Normalisierung des Intermediärstoffwechsels von Diabetikern bei. Für Zink ist außerdem bekannt, dass es bei Diabetikern meist defizitär ist und ein Zusammenhang zwischen Zink, dem Glukosestoffwechsel und Insulin besteht. Einen solchen Zusammenhang gibt es gleichermaßen zwischen dem Glukosestoffwechsel und der Chromversorgung.

Dementsprechend enthält bei einer alternativen, besonders bevorzugten erfindungsgemäßen Verwendung die Zusammensetzung zusätzlich in der wässrigen Phase Zink, Chrom und/oder Mangan. Als die jeweiligen Salze dieser Spurenelemente kommen alle in der Richtlinie 2001/15/EG vom 15. 02 2001 und dementsprechend in der Anlage 2, Liste A zu § 7 Abs. 1 Satz 1 Nr.1, Abs. 2 der deutschen Diät-VO i. d. F. vom 21. 12. 2001 aufgeführten chemischen Verbindungen in Frage, die als Zusatzstoffe für besondere ernährungsphysiologische und diätetische Zwecke im Rahmen von Diätplänen für eine besondere Ernährung bzw. für besondere Ernährungszwecke zulässig sind. Vorzugsweise handelt es sich erfindungsgemäß dabei um solche, die sich durch eine hohe Wasserlöslichkeit auszeichnen und die in der Verwendung nicht prooxidativ wirken. Besonders geeignete Konzentrationen sind 0,00225 bis 0,015 g Zink; 0,03 bis 0,1 mg Chrom und 0,002 bis 0,005 g Mangan in der wässrigen Phase von 35 bis 40 g der eine Fettphase von 60 bis 65 g und damit eine Gesamtmasse von 100 g aufweisenden Endprodukte.

In der am meisten bevorzugten Ausführungsform der erfindungsgemäßen Verwendung hat die Zusammensetzung (bzw. das diätetische Lebensmittel) zur ergänzenden bilanzierten Ernährung/diätetischen Behandlung von Diabetes mellitus in Form eines Streichfettes (Margarine) einen Fettgehalt von 65 % und pro 100 g folgende Zusammensetzung: Energiegehalt : 558 kcal; gesättigte Fettsäuren : 13 g, davon mittelkettige Triglyceride (MCT) 10 g; einfach ungesättigte Fettsäuren (Ölsäure) : 29 g; mehrfach ungesättigte Fettsäuren: 19 g, davon Linolsäure (Omega-6) 14 g, alpha-Linolensäure (Omega-3) 4,25 g, Eicosapentaen- + Docosahexaensäure (Omega-3) 0,75 g; Energie-%-Quotient Omega-6- zu Omega-3-Fettsäuren = 2,9 : 1; Vitamin D₃ : 150 I.E.; Vitamin A : 690 µg; RRR-α-Tocopherol (Vitamin E) : 100 mg; Vitamin C : 200 mg; Vitamin B₁ : 4 mg; Vitamin B₂ :4,5 mg; Vitamin B₆ : 5 mg; Niacin : 53 mg; Folsäure : 1,3 mg; Vitamin B₁₂ :10 µg; β-Carotin :8 µg; Natrium < 0,05 g; frei von Trans-Fettsäuren aus gehärteten Fetten.

### Beispiel

### Zusammensetzung und Herstellung einer Margarine zur ergänzenden bilanzierten Ernährung/diätetischen Behandlung von Erwachsenen und Heranwachsenden mit Diabetes mellitus

Die Zusammensetzung (bzw. das diätetische Lebensmittel) für die erfindungsgemäße Verwendung wird aufgrund seiner Hauptbestandteile bevorzugt in Form von Margarinen und/oder Speiseölen in den Verkehr gebracht.

Als Margarine enthält das erfindungsgemäße diätetische Lebensmittel in der Wasserphase vorzugsweise entweder etwa 20 oder 35 % Wasser und in der Fettphase dementsprechend entweder 80 oder 65 % Fett. Als Speiseöl enthält das erfindungsgemäße Lebensmittel insgesamt etwa 99,5% Fett; dieses besteht aus einer Mischung von Pflanzenölen und MCT-Fett.

Rezepturbeispiele sind in den nachfolgenden beiden Tabellen 1 und 2 angegeben.

**Tabelle 1**

| **Rezepturen für Diabetiker-Fette** | | | | | |
|---|---|---|---|---|---|
| **Margarine mit 80% Fett** | | **Margarine mit 65% Fett** | | **Öl** | |
| Rapsöl | 35,0 g | Rapsöl | 45,0 g | Rapsöl | 69,43 g |
| MCT-Fett | 18,0 g | MCT-Fett | 10,0 g | MCT-Öl | 15,0 g |
| Olivenöl | 13,0 g | | | | |
| Sonnenblumenöl | 10,0 g | Sonnenblumenöl | 7,0 g | Sonnenblumenöl | 15,0 g |
| Fischölkonzentrat^{a} | 3,0 g | Fischölkonzentrat^{a} | 3,0 g | | |
| Retinylpalmitat | 0,001 g | Retinylpalmitat | 0,0011 g | | |
| RRR-α-Tocopheryl-acetat | 0,10 g | RRR-α-Tocopheryl-acetat | 0,10 g | RRR-α-Tocopheryl-acetat | 0,10 g |
| Ascorbylpalmitat | 0,470 g | Ascorbylpalmitat | 0,470 g | Ascorbylpalmitat | 0,470 g |
| Mono- u. Diglyceride von Speisefettsäuren | 0,331 g | Mono- u. Diglyceride von Speisefettsäuren | 0,6 g | | |
| Cholecalciferol | 150 I.E. | Cholecalciferol | 150 I.E. | | |
| β-Carotin^{b} | 0,008 mg | β-Carotin^{b} | 0,0065 mg | | |
| Butteraroma öllöslich | 0,090 g | Butteraroma öllöslich | 0,001 g | | |
| Speisesalz^{d} | 0,10 g | Speisesalz | 0,10 g | | |
| Zitronensäure | 0,005 g | Zitronensäure | 0,042 g | | |
| Butteraroma wasserlöslich | 0,005 g | Butteraroma wasserlöslich^{c} | q.s. | | |

| | | | | | |
|---|---|---|---|---|---|
| ^{a}Hochgereinigtes, mit RRR-α-Tocopherol und Ascorbylpalmitat stabiliertes Fischölkonzentrat, das mindestens 30 % langkettige Omega-3-Polyalkenfettsäuren enthält. | | | | | |
| ^{b}vorzugsweise mit einer höchstens 30 %igen Suspension | | | | | |
| ^{c}falls geschmacklich erforderlich; | | | | | |
| ^{d}Gehalt an Kupfer und Eisen unter 1 part per million (ppm) | | | | | |

**Tabelle 2**

| **Margarine mit 80% Fett** | | **Margarine mit 65% Fett** | |
|---|---|---|---|
| Wässrige Lösung von B-Vitaminen mit | 19,89 g mit | Wässrige Lösung von B-Vitaminen mit | 33,6819 g mit |
| Thiaminmononitrat/L | 220 mg | Thiaminmononitrat/L | 130 mg |
| Riboflavin-5'-phosphat-Natrium/L | 310 mg | Riboflavin-5'-phosphat-Natrium/L | 185 mg |
| Pyridoxinhydrochlorid/L | 305 mg | Pyridoxinhydrochlorid/L | 180 mg |
| Nikotinamid/L | 2665 mg | Nikotinamid/L | 1575 mg |
| Folsäure/L | 65 mg | Folsäure/L | 40 mg |
| Cyanocobalamin/L | 0,5 mg | Cyanocobalamin/L | 0,3 mg |

### Herstellung

### (a) Fettphase

Das MCT-Fett wird, gegebenenfalls mit weiteren, vornehmlich mittelkettige Triglyceride enthaltenden natürlichen Fetten, geschmolzen, wobei die Temperatur 60° C nicht überschreiten darf. Dann werden bei derselben Temperatur die Pflanzenöle beigefügt. Das Fischölkonzentrat und die anderen fettlöslichen Ingredienzien werden dem flüssigen Gemisch erst unmittelbar vor dem Homogenisieren zugegeben.

### (b) Wasserphase

Alle wasserlöslichen Zutaten werden in deionisiertem und entlüftetem Wasser gelöst, und die Lösung wird pasteurisiert.

Die wässrige Phase wird dann bei 40 bis 50°C langsam der Fettphase beigegeben, und mit einer in hoher Geschwindigkeit rotierenden Messerwelle wird eine homogene Emulsion gebildet. Die Kristallisation und das Kneten zu einem streichfähigen Produkt erfolgen in einer bei der Margarineherstellung üblichen Weise mit einem aus A- und B-Units (Kratzbühler und Ruherohr) bestehenden Votator.

Die Abpackung wird bei 15° C vorgenommen, und das Erzeugnis wird an einem kühlen Ort gelagert, gegebenenfalls gefroren (Tiefkühltruhe). Die Haltbarkeit bei 4°C beträgt in der Regel 3 Monate, unter besonders guten Umständen (gefroren, kein Zugang von Luftsauerstoff) auch 6 Monate und länger.

## Patentansprüche

1. Verwendung von mittelkettigen Triglyceriden oder einer mittelkettige Triglyceride enthaltenden Zusammensetzung zur diätetischen Therapie von Diabetes mellitus.

2. Verwendung nach Anspruch 1, wobei die Zusammensetzung in der Fettphase enthält:
(a) 10 bis 30% mittelkettige Triglyceride;
(b) mindestens eine einfach ungesättigte Fettsäure, und/oder
(c) Linolsäure; und/oder
(d) α-Linolensäure.

3. Verwendung nach Anspruch 2, wobei die einfach ungesättigte Fettsäure Ölsäure ist.

4. Verwendung nach Anspruch 3, wobei die Zusammensetzung 20 bis 60 % Ölsäure als einfach ungesättigtes Triglycerid enthält.

5. Verwendung nach einem der Ansprüche 2 bis 4, wobei die Zusammensetzung 10 bis 35 % Linolsäure als zweifach ungesättigtes Triglycerid enthält.

6. Verwendung nach einem der Ansprüche 2 bis 5, wobei die Zusammensetzung 3 bis 10 % alpha-Linolensäure als dreifach ungesättigtes Triglycerid enthält.

7. Verwendung nach einem der Ansprüche 1 bis 6, wobei die Zusammensetzung in der Fettphase weiterhin Eicosapentaensäure und/oder Docosahexaensäure als mehrfach ungesättigte Triglyceride enthält.

8. Verwendung nach einem der Ansprüche 1 bis 7, wobei die Zusammensetzung 0,5 bis 2 % Eicosapentaensäure und/oder Docosahexaensäure enthält.

9. Verwendung nach einem der Ansprüche 1 bis 8, wobei der Gehalt an gesättigten langkettigen Triglyceriden in der Zusammensetzung höchstens 6% beträgt.

10. Verwendung nach einem der Ansprüche 1 bis 9, wobei die Zusammensetzung in der Fettphase folgendermaßen ist :
| | |
|---|---|
| (a) Mittelkettige Triglyceride | 10 bis 30%; |
| (b) Gesättigte langkettige Triglyceride | 0,5 bis 6%; |
| (c) Ölsäure | 20 bis 60%; |
| (d) Linolsäure | 10 bis 35%; |
| (e) alpha-Linolensäure | 3 bis 10%; und |
| (f) Eicosapentaensäure und/oder Docosahexaensäure | 0,5 bis 2%. |

11. Verwendung nach einem der Ansprüche 1 bis 10, wobei die Fettphase außerdem als Emulgatoren Mono- und Diglyceride von Speisefettsäuren, fettlösliche Vitamine, β-Carotin, Butteraromen und/oder in Hinsicht auf die hochungesättigten Fettsäuren geeignete würzende und antioxidativ wirkende Aromen enthält.

12. Verwendung nach Anspruch 11, wobei die fettlöslichen Vitamine die Vitamine A, D, E und/oder Vitamin C in Form von Ascorbylpalmitat sind.

13. Verwendung nach Anspruch 12, wobei die Fettphase 0,0002 bis 0,002 g Retinylpalmitat und/oder 1 bis 5 µg (40-200 I.E.)Vitamin D₃ und/oder 0,02 bis 0,2 g natürliches Vitamin E in Form von RRR-α-Tocopherylacetat und/oder 0,06 bis 0,6 g Ascorbylpalmitat enthält.

14. Verwendung nach einem der Ansprüche 1 bis 13, wobei (a) die Fettphase etwa 80 % und die Wasserphase etwa 20 % oder (b) die Fettphase etwa 60 bis 65 % und die Wasserphase 35 bis 40% betragen.

15. Verwendung nach einem der Ansprüche 1 bis 14, wobei die Wasserphase die Vitamine B₆, B₁₂ und/oder Folsäure enthält.

16. Verwendung nach Anspruch 15, wobei die Wasserphase außerdem die Vitamine C, B₁, B₂ und/oder Niacin enthält.

17. Verwendung nach Anspruch 16, wobei die Zusammensetzung 0,01 bis 0,25 g Vitamin C und/oder 0,0005 bis 0,005 g Vitamin B₁ und/oder 0,0006 mg bis 0,006 g Vitamin B₂ und/oder 0,0007 bis 0,007 g Vitamin B₆ und/oder 0,0015 bis 0,015 mg Vitamin B₁₂ und/oder 0,007 bis 0,070 g Niacin (Nikotinamid) und/oder 0,0002 bis 0,002 g Folsäure enthält.

18. Verwendung nach einem der Ansprüche 1 bis 6, 9 und 14 bis 17, wobei die wässrige Phase der Zusammensetzung Zink, Chrom und/oder Mangan enthält.

19. Verwendung nach Anspruch 18, wobei die Zusammensetzung pro 100 g 0,00225 bis 0,015 g Zink und/oder 0,03 mg bis 0,1 mg Chrom und/oder 0,002 bis 0,005 g Mangan enthält.
